(19) Europäisches Patentamt
European Patent Office
Office européen des brevets

(11) **EP 1 787 588 A1**

(12) **EUROPEAN PATENT APPLICATION**
published in accordance with Art. 158(3) EPC

(43) Date of publication:
**23.05.2007 Bulletin 2007/21**

(51) Int Cl.:
***A61B 10/00*** (2006.01)

(21) Application number: **05774696.8**

(86) International application number:
**PCT/JP2005/015457**

(22) Date of filing: **25.08.2005**

(87) International publication number:
**WO 2006/022342 (02.03.2006 Gazette 2006/09)**

(84) Designated Contracting States:
**DE FR GB**

(30) Priority: **26.08.2004 JP 2004246293**

(71) Applicants:
• **Nippon Telegraph and Telephone Company**
**Tokyo 100-8116 (JP)**
• **School Juridical Person Kitasato Gakuen**
**Minato-ku**
**Tokyo, 1088641 (JP)**

(72) Inventors:
• **Ohbayashi, Kohji**
**The Kitasato Gakuen Foundation**
**Sagamihara-shi, Kanagawa 2288555 (JP)**

• **Shimizu, Kimiya**
**The Kitasato Gakuen Foundation**
**Sagamihara-shi, Kanagawa 2288555 (JP)**
• **Miyazawa, Takeo**
**NTT Intellectual Property Center**
**Musashino-shi, Tokyo 1808585 (JP)**
• **Yoshimura, Ryoko**
**NTT Intellectual Property Center**
**Musashino-shi, Tokyo 1808585 (JP)**

(74) Representative: **Turner, James Arthur**
**D. Young & Co.**
**120 Holborn**
**London EC1N 2DY (GB)**

(54) **TISSUE MEASURING OPTICAL INTERFERENCE TOMOGRAPHY-USE LIGHT PRODUCING DEVICE AND TISSUE MEASURING OPTICAL INTERFERENCE TOMOGRAPHY DEVICE**

(57) It has strongly been desired that the shape of the entire vicinity of a corner angle consisting of a sclera, a cornea and an iris be sufficiently measured with a clear tomographic image. A variable-wavelength light producing device (11), which can emit a light having a wavelength in a 1.53-1.85 $\mu$m region while switching it, has the interference-capable distance of the light in the air of at least 1.4 mm, and can discontinuously switch the wavelength of the light at a wave number interval of up to $1.2 \times 10^{-3}$ $\mu$m$^{-1}$ is used to constitute an eye measuring optical interference tomographic device.

FIG. 1

EP 1 787 588 A1

**Description**

TECHNICAL FIELD

The present invention relates to a tissue measuring optical coherence tomography-use light source and a tissue measuring optical coherence tomography system, and more particularly to a system which is extremely effective when used to obtain a tomographic image of an anterior eye portion in order to examine an eye.

BACKRGOUND ART

Ultrasound Biomicroscopy (UBM), developed by Pavlin et al. in 1990, made it possible to observe the anterior eye portion (the corner angle, the rear surface of the iris, the ciliary body, and so on), which up to that point could not be observed using optical methods. In UBM, the eye is irradiated with ultrasonic waves and the reflected waves generated as a result are analyzed to obtain a tomographic image of the anterior eye portion. UBM has already been put to practical use in the diagnosis of angle-closure glaucoma, ciliary body and lens disorders, intraocular inflammatory disorders, and so on, and is particularly effective for observing the shape of the corner angle in angle-closure glaucoma. Accordingly, UBM is useful for determining an indication for laser iridotomy.

Corner angle observation has also been attempted using an Optical Coherence Tomography system (OCT system) (see Non-Patent Document 1 and so on below, for example). With OCT, a tomographic image of an organ can be observed at a resolution between 10 to 20 $\mu$m, and hence OCT has been employed in medical institutions for observing the retina (see Non-Patent Document 2 and so on below, for example). In a practical application of OCT, a tomographic image of the retina is captured by illuminating the retina with measuring light through transparent tissue such as the cornea, lens, or vitreous body, and thus the retina is observed. In contrast, observation of the corner angle using OCT has only been performed in experiments, and clear images have not been obtained. Furthermore, it has not been acknowledged that the burden on a patient can be greatly released by realizing a corner angle diagnosis system employing an OCT method (see the description in the section "Effects of the Invention" for detail). Hence, a corner angle diagnosis system using OCT has never been developed.

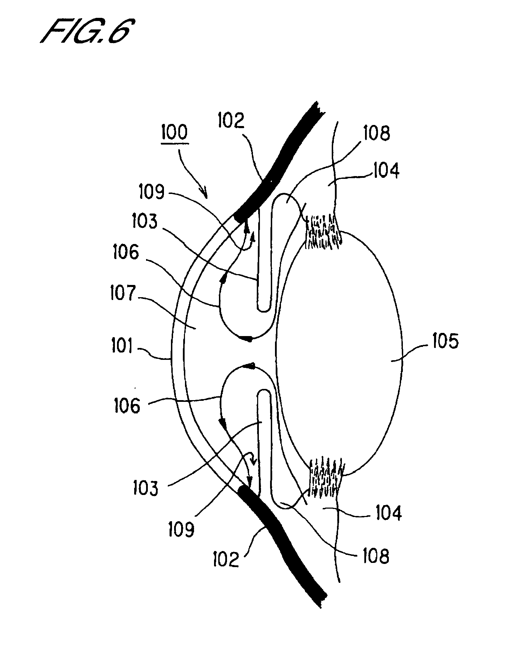

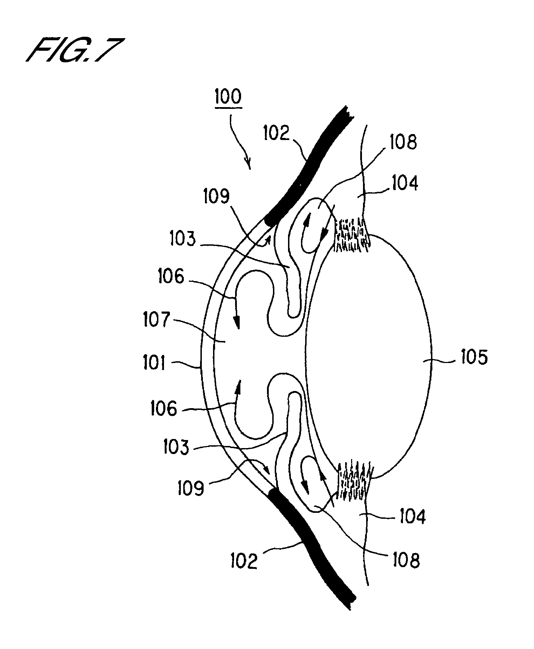

Note that Fig. 6 shows the schematic structure of an eye, and Fig. 7 shows the schematic structure of an eye suffering from angle-closure glaucoma.

Patent Document 1: U.S. Patent Specification No. 4,896,325.

Non-Patent Document 1: Edited by Brett E. Bouma et al., Handbook of Optical Coherence Tomography, (USA), Marcel Dekker Inc., 2002, p. 498 to 500.

Non-Patent Document 2: Chan Kin Pui, "Microscopic diagnostics using optical coherence tomography for clinical applications", Optronics (in Japanese), Optronics Corp., July 10, 2002, No. 247, p. 179 to 183.

Non-Patent Document 3: Yuzo YOSHIKUNI, "Developmental trends of wavelength tunable lasers and expectations for system applications", Oyo Buturi (in Japanse), Applied Physics Scientific Society, 2002, Volume 71, Number 11, p. 1362 to 1366.

Non-Patent Document 4: Edited by Brett E. Bouma et al., Handbook of Optical Coherence Tomography, (USA), Marcel Dekker Inc., 2002, p. 364 to 367.

Non-Patent Document 5: Choi Dong Hak et al., "High speed, high resolution OFDR-OCT using SSG-DBR laser", 28th Optical Symposium Lecture Handbook (in Japanese), The Optical Society of Japan, June 19, 2003, p. 39 to 40.,

Non-Patent Document 6: Masamitsu HARUNA, "Tissue measuring and tomographic imaging using low coherent optical interference", Oyo Kogaku(in Japanese), 2003, Volume 2, p. 1 to 6.

DISCLOSURE OF THE INVENTION

PROBLEMS TO BE SOLVED BY THE INVENTION

However, in the aforementioned UBM, a contact type device is used, and therefore infection, mechanical invasion, or the like may occur. Further, to transmit the ultrasonic waves to the eye efficiently, the eye is covered with an instrument and the space therebetween is filled with water to achieve acoustic impedance matching. Hence, the eye may be held down by the instrument such that the eye deforms, and moreover, measurements must be taken in a face-up position to ensure that the water does not leak. Therefore, in UBM, measurement is complicated and a heavy burden is placed on the patient.

On the other hand, a conventional OCT system such as that described above is a non-contact type device, and therefore problems such as those found in UBM described above do not occur. However, it is difficult to measure a corner angle consisting of an opaque sclera, cornea, and iris, since they are strong scatterer. An attempt has been made to measure the corner angle using light in a longer waveband (1.3$\mu$m) than a typically used waveband (0.8$\mu$m), but in so doing, it is difficult to obtain a clear tomographic image, and the measurement range does not extend to the rear surface of the iris. Moreover, measurement must be completed in a short time period to avoid image disturbance caused by eye movement, and as a result, the measurement range in the horizontal direction narrows, making it difficult to obtain a sufficient measurement of the shape of the entire vicinity of the corner angle, consisting of the sclera, cornea, and iris, which is required to diagnose glaucoma.

Incidentally, OCT includes three main methods, namely OCDR (Optical Coherence Domain Reflectometry), FD (Frequency Domain), and OFDR (Optical Frequency Domain Reflectometry) (see Non-Patent Document 5 and so on above, for example). In the OCDR method, a Superluminescent Diode (SLD) is used as a light source, and light emitted therefrom is input into an interferometer, whereby depth direction information is obtained by varying the optical path length of a reference optical path. Meanwhile, in the FD method, a similar SLD to that of the OCDR method is used as the light source, but the optical path length of the reference optical path remains fixed, and depth direction information is obtained by subjecting an optical spectrum, obtained by dispersing interference light, to Fourier transform. In contrast, the OFDR method uses a wavelength tunable light source as the light source, and obtains depth direction information by subjecting an interference light spectrum obtained by varying the wave number of the light emitted from the light source to Fourier transform.

In practical applications, only OCT systems employing the OCDR-OCT method exist. In an OCT system using the OCDR-OCT method, a reference mirror for varying the optical path length of the reference light must be subjected to a mechanical scan, and since the mechanical scan determines the measurement speed, high speed measurement is difficult. Hence, when measuring tissue such as an eye, which is difficult to maintain in a stationary state, the horizontal direction measurement range becomes narrow, and the measurement range in the depth direction of the tomographic image is limited to a narrow range of approximately 1 to 2mm. A measurement range of at least 3mm or thereabouts is necessary to measure the anterior eye portion, and this narrowing of the measurement range accompanying movement of the tissue makes it difficult to observe an entire image of the anterior eye portion using the OCDR-OCT method.

On the other hand, the FD method does not require a mirror scan, and therefore high speed measurement is possible. Accordingly, narrowing of the measurement range due to movement of the tissue does not pose a problem. However, the depth direction measurement range is fixed (at approximately 2.5mm) by the resolution of a spectroscope used for spectrometry, and hence it is difficult to observe the entire image of the anterior eye portion sufficiently using the FD method.

In addition to measurement of a human anterior eye portion, observation of the eyes of small experimental animals is extremely important for the development of new medicines. When observing the eyes of a small experimental animal, a depth direction measurement range of 1mm or more is sufficient. A depth direction measurement range of 1mm or more is also sufficient for measuring the eyes of a human infant. With this depth direction measurement range, it may be considered possible to perform measurement using either OCDR-OCT or FD-OCT. Unlike an adult human, however, it is difficult for these measurement subjects to keep their eyes stationary in accordance with the instructions of the measurer, and therefore a video rate tomographic image or continuous high speed images corresponding thereto must be taken. With OCDR-OCT, however, it is difficult to perform high speed imaging such as video rate imaging, and although video rate imaging can be implemented with FD-OCT due to the absence of moving parts, a spectroscopy instrument is required, making the system complicated.

As described above, there is strong demand for a novel eye measuring system or measuring method that is easy to operate, does not place a burden on the patient, and is capable of measuring whole the eye or the anterior eye portion, in particular the shape of the entire vicinity of the corner angle, consisting of the sclera, the cornea, and the iris, with a clear tomographic image. There is also demand for a system that can measure a video rate imaging or the like of a clear tomographic image of the eyes of a small experimental animal or an infant.

MEANS FOR SOLVING THE PROBLEMS

To solve the problems described above, the first invention is a tissue measuring optical coherence tomography-use light source characterized in being capable of emitting light in a wavelength region of 1.53 to 1.85$\mu$m.

The second invention, which pertains to the first invention, is a tissue measuring optical coherence tomography-use light source characterized in being capable of emitting this light while switching the wavelength of the light.

The third invention is a tissue measuring optical coherence tomography-use light source characterized in being capable of emitting light having an coherence length in air of 1.4mm or more while switching the wavelength of the light discontinuously in a wave number interval of up to $1.2 \times 10^{-3} \mu m^{-1}$.

The fourth invention, which pertains to the second invention, is a tissue measuring optical coherence tomography-use light source characterized in that the coherence length in air of the light is 1.4mm or more, and in being capable of switching the wavelength of the light discontinuously in a wave number interval of up to $1.2 \times 10^{-3} \mu m^{-1}$.

The fifth invention, which pertains to one of the second to fourth inventions, is a tissue measuring optical coherence tomography-use light source characterized in being capable of emitting the light while switching the wavelength of the light stepwise.

The sixth invention, which pertains to one of the second to fifth inventions, is a tissue measuring optical coherence tomography-use light source characterized in that a light source for producing the light is a wavelength tunable semiconductor laser.

The seventh invention, which pertains to the sixth invention, is a tissue measuring optical coherence tomography-use light source characterized in that the tunable wavelength tunable semiconductor laser is one of a superstructure grating distributed Bragg reflector semiconductor laser, a sampled grating distributed Bragg reflector semiconductor laser, and a grating coupler sampled reflector laser.

The eighth invention is a tissue measuring optical coherence tomography system characterized in comprising the tissue measuring optical coherence tomography-use light source of the first invention.

The ninth invention is a tissue measuring optical coherence tomography system characterized in comprising: wavelength tunable light producing means having the tissue measuring optical coherence tomography-use light source according to one of the second to seventh inventions as a light source; main splitting means for splitting light produced by the wavelength tunable light producing means into measuring light and reference light; measuring light illuminating means for illuminating the measuring light split by the main splitting means onto a target tissue while scanning the tissue; signal light collecting means for collecting signal light illuminated onto the tissue and reflected or backscattered thereby; combining means for combining the signal light colllected by the signal light collecting means and the reference light split by the main splitting means; and calculation control means for controlling the wavelength tunable light producing means such that the light is produced by the wavelength tunable light producing means at a target wavelength, and determining a tomographic image of the tissue on the basis of the wavelength of the light produced by the wavelength tunable light producing means and an intensity of the light combined by the combining means.

The tenth invention, which pertains to the ninth invention, is a tissue measuring optical coherence tomography system characterized in that the main splitting means and the combining means serve together as main splitting/combining means.

The eleventh invention, which pertains to the ninth or tenth invention, is a tissue measuring optical coherence tomography system characterized in that the measuring light illuminating means and the signal lightcollecting means serve together as illuminating/collecting means.

The twelfth invention, which pertains to one of the eighth to eleventh inventions, is a tissue measuring optical coherence tomography system characterized in that the tissue has a water content of at least 60% by weight.

The thirteenth invention, which pertains to the twelfth invention, is a tissue measuring optical coherence tomography system characterized in that the tissue is an eye.

The fourteenth invention, which pertains to the thirteenth invention, is a tissue measuring optical coherence tomography characterized in that an anterior eye portion of the eye is measured.

The fifteenth invention, which pertains to the thirteenth or fourteenth invention, is a tissue measuring optical coherence tomography system characterized in comprising fixing/supporting means for fixedly supporting a face of a test subject while the test subject is in a sitting position and the eye remains oriented in a horizontal direction.

EFFECTS OF THE INVENTION

According to the first and the third to seventh inventions, light in a wavelength region of 1.53 to 1.85μm is used as the measuring light, and therefore the effects of light scattering can be reduced while suppressing the effects of light absorption in water. Therefore, a clear tomographic image of tissue on the rear of a scatterer such as the sclera or iris of the eye, for example, can be captured.

Further, by applying the second to seventh inventions to an optical frequency domain reflectometry OCT method (OFDR-OCT method), a high speed operation is possible, and therefore image distortion caused by movement of the measurement subject does not occur even when a tomographic image is captured in a wide range (the horizontal direction and depth direction). More specifically, during an eye examination, for example, it is important for the depth direction measurement range to be as large as possible, but in the OFDR-OCT method, the measurement depth relative to the eye, which has an average refractive index of 1.35, can be set at 3mm by setting the wave number interval within the wavelength sweep time to $3.9 \times 10^{-4} \mu\mathrm{m}^{-1}$ or less, and hence the required measurement depth for measuring the anterior eye portion (the region extending from the front surface of the cornea to the rear surface of the lens), and in particular for measuring the corner angle, can be secured. Furthermore, when measuring the eye of a small experimental animal or an infant, a measurement depth of 1mm, which is required for measuring the anterior eye portion of the measurement subject, can be secured by setting the wave number interval to $1.2 \times 10^{-1} \mu\mathrm{m}^{-1}$ or less, and a video rate tomographic image or high-speed continuous images corresponding thereto can be taken with a simple system constitution.

Furthermore, in the OFDR-OCT method, it is easy to narrow the wave number interval even further, and hence the measurement depth can be set at 10mm. Measurement in such a deep range cannot be implemented easily using other OCT methods.

Further, the OFDR-OCT method has an extremely high sensitivity in comparison with a conventional OCDR-OCT method, and this feature is also highly advantageous for capturing clear tomographic images of tissue on the rear of a scatterer such as the sclera or the like. Note that the high sensitivity of OFDR-OCT is based on an increase in the signal strength of the tomographic image proportionate to the number of wave numbers (or the square thereof) used in the measurement.

According to the sixth and seventh inventions, a wavelength tunable semiconductor laser, or more specifically a superstructure grating distributed Bragg reflector semiconductor laser, which has been developed to a high standard for communication purposes, a sampled grating distributed Bragg reflection semiconductor laser (SG-DBR laser), a grating coupler sampled reflector laser (GCSR laser), or the like is used as a light source for producing light in the aforementioned wavelength region in the OFDR-OCT method, and therefore the operation described above can be obtained easily.

The eighth to fifteenth and the seventeenth to nineteenth inventions exhibit identical effects to the first to seventh inventions cited thereby.

Further, according to the twelfth to fifteenth inventions, the effects of light scattering can be reduced while suppressing the effects of light absorption in water, and therefore clear tomographic images of tissue having a water content of 60% or more, such as the eye, can be captured. In other words, OCT using light in a wavelength region of 1.53 to 1.85$\mu$m as measuring light is capable of reducing the effects of light scattering can be reduced while suppressing the effects of light absorption in water, and is therefore effective when capturing tomographic images of the eye and other tissue having a large water content (60% or more).

Further, according to the thirteenth to fifteenth inventions, the measuring light in the aforementioned wavelength region (1.53 to 1.85$\mu$m) is absorbed in water appropriately, and therefore almost never passes through the vitreous body of the eye, which is constituted by approximately 99% water and has a magnitude of approximately 2cm, to reach the retina, for example. Therefore, the safety of the measuring light in relation to the retina, which is extremely important eye tissue, is very high. Hence, according to the present invention, a clear tomographic image of the shape of the entire vicinity of the corner angle of the eye, for example, consisting of the sclera, cornea, and iris, can be measured safely.

Furthermore, according to the fifteenth invention, the eye can be examined without placing a burden on the patient. More specifically, a non-contact OCT method is used, and therefore the eye tissue can be observed with the test subject in a sitting position such that the eye is oriented in the horizontal direction. This is a superior feature not present in UBM, where measurement must take place in a face-up position. Furthermore, since there is no need to hold the eye down with an instrument, the measurement subject eye does not deform.

According to the sixteenth invention, a pre-existing slit lamp microscope is used, and therefore a reasonably-priced OCT eye examining system can be constructed. More specifically, fixing/supporting means for fixedly supporting the face of the test subject with the test subject in a sitting position such that the eye remains oriented in the horizontal direction are not provided, whereas means for attaching means for illuminating the eye with the measuring light to a slit lamp microscope are provided, and therefore a system which enables construction of an eye-diagnosing optical coherence tomography system can be provided easily. Fixing/supporting means are expensive, and therefore an eye-diagnosing optical coherence tomography system can be constructed at low cost using a pre-existing slit lamp microscope in an ophthalmic surgery. Note that this system may be applied to an optical coherence tomography system using a light source in any wavelength region.

Further, according to the eighteenth and nineteenth inventions, each of the following steps can be realized: a step of illuminating eye tissue with light produced by a tissue measuring optical coherence tomography-use light source; a step of detecting reflection light or backscattered light produced in the interior of the eye tissue using a detector; and a step of generating the depth direction structure of the eye tissue on the basis of detection data detected by the detector using a tissue measuring optical coherence tomography system. Hence, according to the present invention, a method of diagnosing eye tissue can be realized.

BRIEF DESCRIPTION OF THE DRAWINGS

Fig. 1 is a block diagram of an embodiment in which a tissue measuring optical coherence tomography system according to the present invention is applied to an eye measuring optical coherence tomography system.
Fig. 2 is a block diagram of a measuring head shown in Fig. 1.
Fig. 3 is an illustrative view of a method of scanning the wavelength of light emitted from a wavelength tunable light source.
Fig. 4 is an OFDR-OCT image of an eye.
Fig. 5 is a graph showing a relationship between the wavelength of the measuring light in water and an optical absorption coefficient.

Fig. 6 is a schematic structural diagram of an eye.

Fig. 7 is a schematic structural diagram of an eye suffering from angle-closure glaucoma.

Fig. 8 is a tomographic image of an anterior eye portion.

Fig. 9 is a block diagram of means for attaching the measuring head to a slit lamp microscope.

DESCRIPTION OF REFERENCE SYMBOLS

| | |
|---|---|
| 11 | wavelength tunable light source |
| 12 | first coupler |
| 13 | second coupler |
| 14 | aiming light source |
| 15 | optical circulator |
| 16 | third coupler |
| 17 | first differential amplifier |
| 18 | second differential amplifier |
| 19 | photodetector |
| 20 | Logarithmic amplifier |
| 21 | arithmetic calculation control device |
| 22 | display device |
| 40 | measuring head |
| 41 | main body tube |
| 41a | light entrance/exit window |
| 42 | collimator lens |
| 43 | galvanometer mirror |
| 44 | focusing lens |
| 50 | support |
| 51 | movable stage |
| 52, 53 | support arm |
| 60 | microscope |
| 100 | eye |
| 101 | cornea |
| 102 | sclera |
| 103 | iris |
| 104 | ciliary body |
| 105 | lens |
| 106 | aqueous humor |
| 107 | anterior sac |
| 108 | posterior sac |
| 109 | corner angle |
| 201 | means for attaching measuring head to slit lamp microscope |
| 202 | flat plate |
| 203 | hole |
| 204 | space |

BEST MODE FOR CARRYING OUT THE INVENTION

[Principles]

An OCT method diagnostic system used as a method of capturing a tomographic image of an eye mainly observes the retina, for example, and has not yet been used to diagnose the anterior eye portion. When the anterior eye portion is measured by this system, it is impossible to observe the corner angle, a part of which is hidden in the shadow of the sclera, or the ciliary body on the rear surface of the iris.

In a practical application of an OCT system, a Superluminescent Diode (SLD) having a center wavelength of $0.83\mu$m is used as a light source. Meanwhile, an attempt to measure the corner angle using an SLD with a center wavelength of $1.31\mu$m to reduce the effects of light scattering and hence obtain a clear image has been reported. When diagnosing angle-closure glaucoma, the entire shape of the cornea, sclera, and iris in the vicinity of the corner angle is observed using UBM. However, an OCT method image measured using light with a center wavelength of $1.31\mu m$ is unclear even when the measurement wavelength is increased to reduce the effects of light scattering. Moreover, only the front surface part of the iris can be observed, and it is impossible to observe the entire iris. It has been conjectured that the reason for this is that the measuring light is scattered by a scatterer such as the sclera or iris, and therefore the measuring light cannot reach the base part of the iris, which is hidden behind the sclera, or the rear surface of the iris.

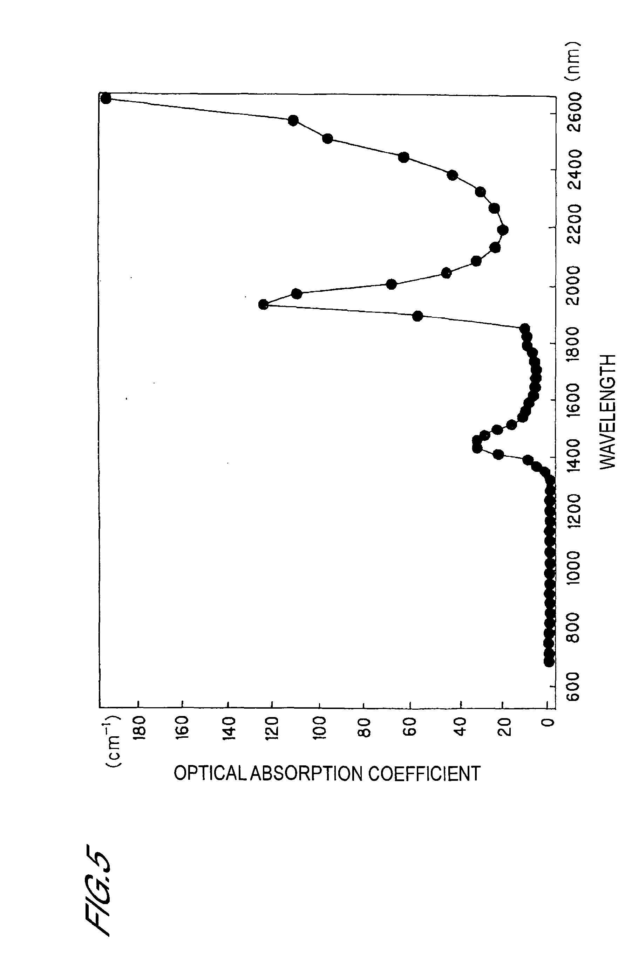

Hence, when light having a longer center wavelength than $1.31\mu m$ is used, scattering of the measuring light is conceivably reduced, but since the main component of the measurement subject tissue is water and the optical absorption coefficient relative to water increases greatly when the center wavelength reaches or exceeds $1.4\mu m$, as shown in Fig. 5, up to this point it has been considered pointless to perform OCT measurement on tissue using a measurement wavelength with a center wavelength of $1.4\mu m$ or more.

However, as a result of keen investigation by the present inventors, it was found that with light having a wavelength region between 1.53 and $1.85\mu m$, the effects of absorption by water are substantially negligible, and that clear images are obtained. It is conjectured that the reason for this is that light in a wavelength region between 1.53 and $1.85\mu m$ has a comparatively small water absorption coefficient of $10cm^{-1}$, and therefore the effects of absorption by water are unlikely to become obvious. Moreover, the intensity of light scattering on tissue decreases rapidly as the wavelength increases, and therefore scattering decreases when the wavelength of the measuring light is increased, enabling the measuring light to penetrate deeply.

Hence, when light in a wavelength region between 1.53 and $1.85\mu m$ is used as the measuring light, the shape of the entire vicinity of the corner angle, consisting of the sclera, cornea, and iris, can be measured sufficiently with a clear tomographic image.

Note that the wavelength region of the measuring light is more preferably between 1.58 and $1.80\mu m$, and even more preferably between 1.68 and $1.70\mu m$. To suppress absorption by water to the greatest extent possible, the wavelength region of the measuring light becomes gradually more preferable in the following order: 1.59 to $1.79\mu m$, 1.60 to $1.78\mu m$, 1.61 to $1.77\mu m$, 1.62 to $1.76\mu m$, 1.63 to $1.75\mu m$. On the other hand, in terms of the availability, reliability, and so on of the light source, a $1.5\mu m$ band (for example, a region covered by the C band (1.530 to $1.565\mu m$) or the L band (1.565 to $1.610\mu m$)), which is a waveband developed for communication purposes, is preferable as the wavelength region of the measuring light, and more specifically, the wavelength region of the measuring light becomes gradually more preferable in the following order: 1.53 to $1.610\mu m$, 1.53 to $1.59\mu m$, 1.53 to $1.57\mu m$, 1.54 to $1.56\mu m$.

There are further advantages to using light in the aforementioned wavelength region as measuring light in addition to those cited above. The absorption coefficient relative to water of the aforementioned wavelength region is comparatively small, i.e. $10cm^{-1}$, but even so, the measuring light almost never passes through the vitreous body, which is 99% water and has a diameter of approximately 2cm, to reach the retina, and therefore the safety of the measuring light in relation to the retina, which is extremely important eye tissue, is very high.

In short, OCT using light in a wavelength region of 1.53 to $1.85\mu m$ as measuring light is suitable for tomographic image measurement of an entire region extending from the front surface to the rear surface of the iris, and also suitable for tomographic image measurement of an entire region extending from the front surface to the rear surface of the sclera. Accordingly, OCT using light in a wavelength region of 1.53 to $1.85\mu m$ as measuring light is suitable for tomographic image measurement of a part or the whole of the anterior eye portion (a region extending from the front surface of the cornea to the rear surface of the lens).

As described above, the wavelength region of the light used as measuring light is a range of 1.53 to $1.85\mu m$, but of course there is no need to use this entire wavelength region alone as measuring light, and light in a partial wavelength region of the region described above, for example 1.53 to $1.57\mu m$, or light in a wavelength region encompassing the entire region described above, for example 1.50 to $1.90\mu m$, may be used as measuring light. In other words, light having a wavelength within a region of 1.53 to $1.85\mu m$ may be used as all or a part of the measuring light. This applies similarly to the various preferred wavelength regions described heretofore.

Note that in conventional OCT using an SLD as a light source, i. e. in an OCDR-OCT method or an FD method, the center wavelength of the light emitted from the SLD used as a light source of an OCDR-OCT system or an FD-OCT system is preferably within a wavelength region of 1.53 to $1.85\mu m$.

Furthermore, since the OCT method is a non-contact method, the eye tissue can be observed in a sitting position, as will be described in the following example. This is a superior feature not exhibited by UBM, in which measurement must be performed in a face-up position. Moreover, since the eye does not have to be held down by an instrument, the measurement subj ect eye does not deform.

The OCT method is effective not only for measuring the corner angle, but also for measuring foreign matter (metal pieces) that has entered the eye and measuring other parts of the anterior eye portion than the corner angle (the rear surface of the iris, the ciliary body, and so on). Measurement of the lens is also possible, and therefore the OCT method is also effective in preoperative and postoperative diagnoses of cataract surgery. It is also possible to measure a part of the vitreous body through the anterior eye portion.

As described above, in practical applications, only OCT systems employing the OCDR-OCT method exist. In an OCT system using the OCDR-OCT method, a reference mirror for varying the optical path length of the reference light must be subjected to a mechanical scan, and since the mechanical scan determines the measurement speed, high speed measurement is difficult, and as a result, the measurement range in the depth direction of the tomographic image is limited to a narrow range of approximately 1 to 2mm. In contrast, the OFDR-OCT method developed by the present inventors uses wavelength tunable light, and therefore a mechanical scan of a reference mirror and spectrometry are not required. Hence, high speed measurement is possible, and a measurement range of 3mm or more can be achieved easily. Moreover, the spectrometry performed in the FD method is not required, and therefore the depth direction measurement range is not limited (to 2.5mm or less). Hence, when measuring the eyes of a small experimental animal or an infant, a video rate tomographic image or high-speed continuous images corresponding thereto can be taken with a simple system constitution. The OFDR-OCT method is also advantageous in that the signal strength is between 10 and 1000 times greater than that of other OCT methods.

In the OFDR-OCT method, a depth direction measurement range $\Delta z$ in tissue having a refractive index of n is dependent on a wave number interval $\delta k$ of the light emitted from the light source, as shown in the following Equation (1).

$$\Delta z = \pi/(2n\ \delta k) \qquad\qquad\qquad (1)$$

The average refractive index n of the eye is 1.35, and therefore, by setting the wave number interval of the measuring light to no more than $3.9\times10^{-4}\mu m^{-1}$ in Equation (1), the eye can be measured in a range of 3mm or more. Needless to say, the coherence length of the light source in air must be within the desired measurement range (in the case described above, at least 4.1mm ($3mm\times1.35$)).

To observe the anterior eye portion, including that of a small experimental animal or infant, the wave number interval of the measuring light is preferably set to no more than $1.2\times10^{-3}\mu m^{-1}$ and the coherence length in air is preferably set to no less than 1. 4mm, whereby the measurable range in the depth direction is at least 1mm. However, when the measurable range in the depth direction is set at 2mm or more, the wave number interval of the measuring light is preferably set to no more than $5.8\times10^{-4}\mu m^{-1}$ and the coherence length in air is preferably set to no less than 2.7mm. Further, when the measurable range in the depth direction is set at 4mm or more, the wave number interval of the measuring light is preferably set to no more than $2.9\times10^{-4}\mu m^{-1}$ and the coherence length in air is preferably set to no less than 5.4mm; when the measurable range in the depth direction is set at 5mm or more, the wave number interval of the measuring light is preferably set to no more than $2.3\times10^{-4}\mu m^{-1}$ and the coherence length in air is preferably set to no less than 6.8mm; when the measurable range in the depth direction is set at 6mm or more, the wave number interval of the measuring light is preferably set to no more than $1.9\times10^{-4}\mu m^{-1}$ and the coherence length in air is preferably set to no less than 8.1mm; when the measurable range in the depth direction is set at 9mm or more, the wave number interval of the measuring light is preferably set to no more than $1.3\times10^{-4}\mu m^{-1}$ and the coherence length in air is preferably set to no less than 12mm; and when the measurable range in the depth direction is set at 12mm or more, the wave number interval of the measuring light is preferably set to no more than $9.7\times10^{-5}\mu m^{-1}$ and the coherence length in air is preferably set to no less than 16mm.

Here, the coherence length of the light refers to the full width at half maximum when the intensity (power intensity) of interference light produced by incident light into a Michelson interferometer is measured as a function of the difference in distance length between an optical path split in two (the distance from a beam splitting point to a beam combining point). As regards the wave number interval, the wave number is never singular, and therefore the wave number interval does not include $0\mu m^{-1}$.

In OCT using a wavelength tunable semiconductor laser as a light source, the depth direction measurement range can be widened easily, and due to the high speed of the measurement, a two-dimensional scan of a wide range can also be performed. Therefore, OCT using a wavelength tunable semiconductor laser as a light source is suitable for measuring a tomographic image of the eye (particularly the anterior eye portion). More specifically, OCT using a wavelength tunable semiconductor laser as a light source is suitable for measuring the corner angle, in particular one half side of the anterior eye portion consisting of one entire side of the iris extending from the attachment of the iris and the sclera to the pupil, and the cornea (and sclera) positioned thereabove, and also for measuring the entire anterior eye portion consisting of the iris extending from one attachment of the iris and sclera to the other attachment of the iris and sclera, and the cornea (and sclera) positioned thereabove. Note that the iris is preferably measured up to the rear surface, but glaucoma and the like may be diagnosed without measuring the iris up to the rear surface.

Further, in OCT used light in a wavelength region of 1.53 and 1.85$\mu$m as measuring light, the effects of light scattering can be reduced while suppressing the effects of light absorption by water, and therefore tomographic images of tissue having a large water content other than the eye can also be captured effectively. Here, the water content of representative tissues is illustrated in Table 1 below. As is evident from Table 1, tissue other than bone tissue (including tooth tissue) and fatty tissue has a water content of 60% by weight or more, and therefore tomographic images thereof can be taken favorably with an OCT method using light in the above wavelength region as measuring light. Note that for favorable tomographic images to be captured with an OCT method using light in the above wavelength region as measuring light, the water content of the tissue is preferably no less than 60% by weight, more preferably no less than 70% by weight, and even more preferably no less than 80% by weight.

[Table 1]

| Tissue | Water Content(% by weight) |
|---|---|
| Brain | 74.8 |
| Heart | 79.2 |
| Lung | 79.0 |
| Muscle | 75.6 |
| Blood | 83.0 |
| Liver | 68.3 |
| Kidney | 82.7 |
| Intestine | 74.5 |
| Skin | 72.0 |
| Bone | 22.0 |
| Fat | 10.0 |
| Tooth (Enamel) | 1 - 2 |
| Tooth (Dentine) | 18 |
| Tooth (Cement) | 32 (including Organic 32 Matter) |

A wavelength tunable laser satisfying the conditions described above is easily available, and representative examples thereof include a superstructure grating distributed Bragg reflector wavelength tunable semiconductor laser (SSG-DBR laser) (see Non-Patent Document 3 and so on, for example). It is also believed that a sampled grating distributed Bragg reflector wavelength tunable semiconductor laser (SG-DBR laser) and a GCSR laser satisfy the above conditions (see Non-Patent Document 1 and so on, for example).

[Example]

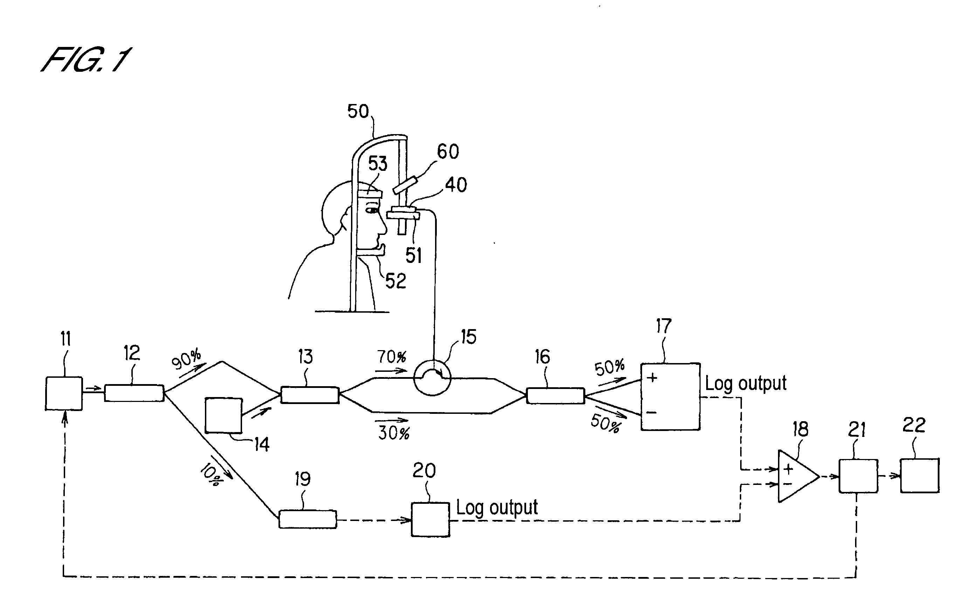

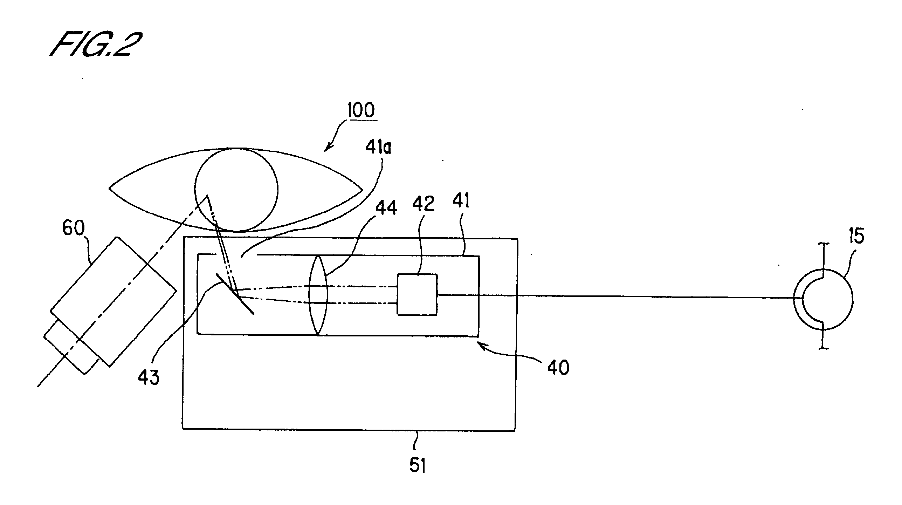

Next, an embodiment of a case in which the tissue measuring optical coherence tomography system according to the present invention is applied to an eye examination will be described on the basis of Figs. 1 and 2. Fig. 1 is a block diagram of an eye measuring optical coherence tomography , and Fig. 2 is a block diagram of a measuring head shown in Fig. 1.

As shown in Fig. 1, a light emission port of a wavelength tunable light source 11, which serves as wavelength tunable light producing means for emitting light while varying the wavelength thereof such as a semiconductor laser light source using a superstructure grating distributed Bragg reflector semiconductor laser (see Non-Patent Document 3 and so on, for example) as a light source and comprising peripheral equipment such as a control system, for example, is optically connected to a light incident port of a first coupler 12 constituted by a directional coupler or the like that splits the light into two (90:10, for example).

A light output port on one side (the 90% split proportion side) of the first coupler 12 is optically connected to a light incident port of a second coupler 13 serving as main splitting means and constituted by a directional coupler or the like that splits the light into two (70:30, for example). A light emission port of an aiming light source 14, which is a visible light source that emits light in a visible region to enable viewing of the irradiation position of the measuring light, is optically connected to the light incident port of the second coupler 13.

A light output port on one side (the 70% split proportion side) of the second coupler 13 is optically connected to a light incident port of an optical circulator 15. A light output port on the other side (the 30% split proportion side) of the second coupler 13 is optically connected to a light incident port of a third coupler 16 serving as combining means and constituted by a directional coupler or the like that splits the light in two (50:50, for example). A light output port of the optical circulator 15 is optically connected to the light incident port of the third coupler 16 and connected to a base end side of a measuring head 40. The measuring head 40 is attached to a movable stage 51 provided on a support 50, and is structured as shown in Fig. 2.

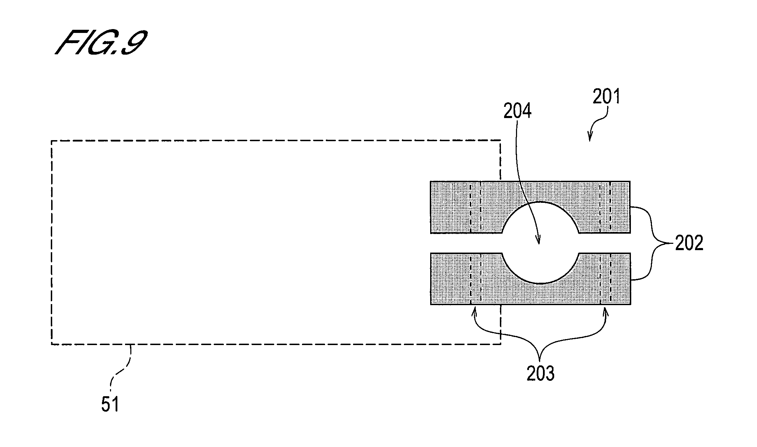

Members including the support 50 for supporting the measuring head, support arms 52, 53 for supporting the face of a test subject in a sitting position, and a microscope 60 for observing the eye of the test subject do not necessarily have to be constructed integrally with other members such as the wavelength tunable light source 11. These members are all provided in a slit lamp microscope used in standard ophthalmic medical treatments, and therefore, by providing another member with means for attaching the measuring head to a slit lamp microscope, a system for attaching the measuring head to a pre-existing slit lamp microscope in an ophthalmic surgery in order to construct an eye diagnosing optical coherence tomography system can be created easily. The support 50 and so on for supporting the measuring head are expensive, and therefore, when a pre-existing slit lamp microscope in an ophthalmic surgery is used, an eye diagnosing optical coherence tomography system can be constructed at low cost. Note that this system may be applied to an optical coherence tomography system using a light source in any wavelength region. Fig. 9 shows an example of means 201 for attaching a measuring head to a slit lamp microscope. The means 201 for attaching a measuring head to a slit lamp microscope are constituted by two flat plates 202. Two rows of holes 203 penetrating the flat plates serve as female screws, and the two flat plates 202 are integrated by fitting male screws (not shown) into the holes 203. A space 204 corresponding to the transverse sectional shape of the support 50 is provided in the center of the integrated attaching means 201. The support 50 is held in the space, and by tightening the aforementioned screws, the attaching means 201 are fixed to the support 50. The movable stage 51 is fixed to a left end of the attaching means 201.

As shown in Fig. 2, the measuring head 40 comprises a main body tube 41 supported on the movable stage 51 of the support arm 50 and formed with a light entrance/exit window 41a in a part of a tip end side peripheral wall thereof, a collimator lens 42 disposed on a base end side of the interior of the main body tube 41 and optically connected to the optical circulator 15, a galvanometer mirror 43 disposed on a tip end side of the interior of the main body tube 41 and capable of a scanning motion enabling modification of the advancement direction of the measuring light, and a focusing lens 44 disposed between the collimator lens 42 and galvanometer mirror 43 in the interior of the main body tube 41. Further, the support 50 is provided with the support arms 52, 53 for fixedly supporting the face of the test subject in a sitting position such that the eyes of the test subject remain oriented in a horizontal direction, and attached with the visual confirmation microscope 60 serving as irradiation position confirming means.

Measuring light that enters the collimator lens 42 in the interior of the main body tube 41 of the measuring head 40 from the optical circulator 15 is formed into parallel beams that converge on the focusing lens 44, whereupon the measuring light exits through the light entrance/exit window 41a of the main body tube 41 via the galvanometer mirror 43 and impinges on an eye 100. The resultant reflected (backscattered) signal light enters the interior of the main body tube 41 through the light entrance/exit window 41a, is reflected by the galvanometer mirror 43, and enters the optical circulator 15 from the base end side of the main body tube 41 via the focusing lens 44 and collimator lens 42.

In this embodiment, the optical circulator 15, measuring head 40, and so on constitute illuminating/collecting means doubling as measuring light illuminating means and signal light collecting means, while the support 50 and so on double as illuminating/collecting means position adjusting means and fixing/supporting means.

As shown in Fig. 1, light output ports on one side and the other side of the third coupler 16 are optically connected to a light incident port of a first differential amplifier 17 having a light detection function. A Log output portion of the first differential amplifier 17 is electrically connected to a Log input portion of a second differential amplifier 18 for performing a correction calculation relating to variation in the strength of the input signal.

Meanwhile, a light output port on the other side (the 10% split proportion side) of the first coupler 12 is optically connected to a light incident port of a photodetector 19. An output portion of the photodetector 19 is electrically connected to an input portion of a logarithmic amplifier 20. A Log output portion of the logarithmic amplifier 20 is electrically connected to the Log input portion of the second differential amplifier 18.

An output portion of the second differential amplifier 18 is electrically connected to an input portion of a calculation control device 21 for synthesizing the coherent interference waveform, or in other words the backscattering intensity distribution (see Non-Patent Document 5 and so on, for example) via an analog/digital converter, not shown in the drawing. An output portion of the calculation control device 21 is electrically connected to an input portion of a display device 22 such as a monitor or printer for displaying a calculation result. The calculation control device 21 is capable of controlling the wavelength tunable light source 11 on the basis of input information.

In this embodiment, the first differential amplifier 17, second differential amplifier 18, photodetector 19, logarithmic amplifier 20, calculation control device 21, display device 22, and so on constitute calculation control means.

Next, a method of measuring the eye using the eye measuring optical coherence tomography system according to this embodiment will be described.

The face of the test subject is fixedly supported by the support arms 52, 53 of the support 50 such that the eyes of the test subject remain oriented in the horizontal direction, whereupon the aiming light source 14 is activated such that viewing light from the aiming light source 14, which is illuminated from the tip end side of the measuring head 40, is visually confirmed by the microscope 60. The movable stage 51 of the support 50 is then adjusted such that a target location of the eye 100 of the test subject is irradiated with the viewing light.

Next, the calculation control device 21 is activated such that measuring light in the target wavelength region (wavelength tunable range: 1.530 to 1.570μm, wave number interval: $2.3 \times 10^{-4}$μm$^{-1}$, spectral frequency width: 10MHz or less, coherence length in air: 13mm or more, or wavelength tunable range: 1.68 to 1.70μm, wave number interval: $9.7 \times 10^{-5}$μm$^{-1}$, spectral frequency width: 10MHz or less, coherence length in air: 13mm or more, where the coherence length in air is calculated from the spectral frequency width using Equation (4) of Non-Patent Document 5) is produced by the wavelength tunable light source 11.

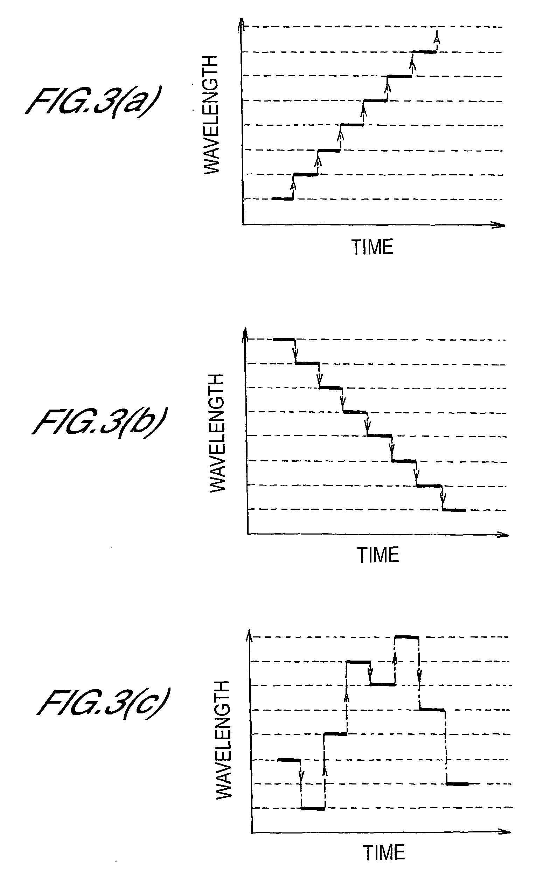

At this time, the measuring light is produced while switching the wave number of the light such that the wave number varies stepwise relative to the sweep time, and the employed switching method (sweeping method) may be such that the wave number is gradually increased, as shown in Fig. 3(a), gradually decreased, as shown in Fig. 3(b), or varied irregularly, as shown in Fig. 3(c). In short, all of a plurality of predetermined wave numbers should be scanned within the measurement period. Note that a special OCT system known as a chirp OCT system, which has a similar system constitution to an OFDR-OCT system, exists in the related art (see Non-Patent Document 4 (p. 365) and so on, for example) for varying the wave number of measuring light produced by a light source in a linear fashion relative to the sweep time, but the present inventors have discovered that a clearer tomographic image can be obtained when measuring light produced by an OFDR-OCT light source is varied stepwise relative to the sweep time. Further, the aforementioned "predetermined wave numbers" are preferably a collection of wave numbers arranged at equal intervals, but the present invention is not limited thereto, and may be applied to a collection of wave numbers arranged at irregular intervals in consideration of the calculation processing that is performed during tomographic image creation, for example.

The light produced by the wavelength tunable light source 11 is split into two (90:10) by the first coupler 12. The light on one side (the 90% side) of the light split into two by the first coupler 12 is split into two by the second coupler 13 (70:30). The light (correction light) on the other side (the 10% side) of the light split into two by the first coupler 12 is transmitted to the optical detector 19.

The light (measuring light) on one side (the 70% side) of the light that is split into two by the second coupler 13 passes through the measuring head 40 via the optical circulator 15 together with the aforementioned viewing light, and is illuminated from the tip end side of the measuring head 40 onto the eye 100 of the test subject, as described above.

The light (signal light) that is illuminated onto and reflected (or backscattered) by the eye 100 re-enters the measuring head 40, as described above, and is transmitted to the third coupler 16 via the optical circulator 15. The light (reference light) on the other side (the 30% side) of the light split into two by the second coupler 13 is transmitted to the third coupler 16 and combined with the signal light.

The light combined by the third coupler 16 is transmitted to the first differential amplifier 17. The first differential amplifier 17 outputs a logrithmic output signal to the second differential amplifier 18. The optical detector 19 converts the light (correction light) on the other side (the 10% side) of the light split into two by the first coupler 12 into an electric signal and outputs the electric signal to the logrithmic amplifier 20. The logrithmic amplifier 20 outputs a logrithmic output signal to the second differential amplifier 18. The second differential amplifier 18 performs an input intensity correction calculation, and then outputs a corresponding information signal to the aforementioned analog/digital converter.

The analog/digital converter converts the input information signal into a digital signal, and outputs this digital signal to the calculation control device 21. The calculation control device 21 performs calculation processing on the basis of the various input information (see Non-Patent Document 5 and so on, for example), determines a coherent interference waveform, or in other words the intensity of the signal light, produces a tomographic image of the eye 100 on the basis of this intensity and so on, and displays the result on the display device 22.

At this time, a scan (Bscan) can be performed in a transverse direction to the optical axis by scanning the galvanometer mirror 43 of the measuring head 40 in a horizontal direction, and hence an entire tomographic image can be produced.

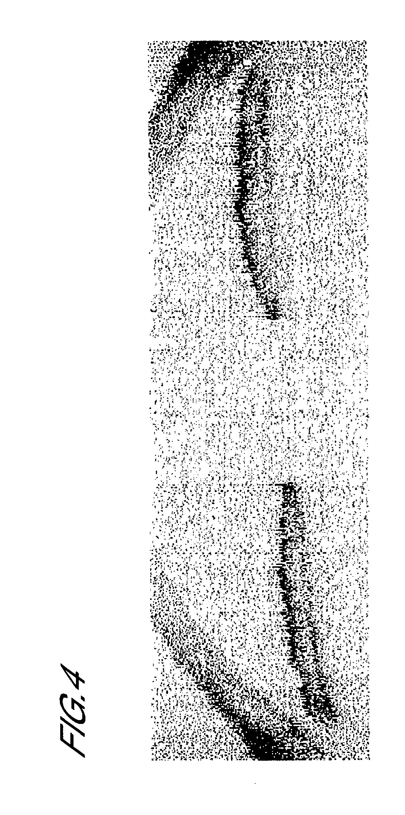

Fig. 4 shows an example of a result of measurement of the eye 100 using the eye measuring optical coherence tomographic system described above (captured at a wavelength tunable range of 1.53 to 1.57μm, a wave number interval of $2.3 \times 10^{-4}$μm$^{-1}$, and an instantaneous spectral frequency width of 10MHz). The time needed to obtain a tomographic image such as that shown in Fig. 4 is no more than one second.

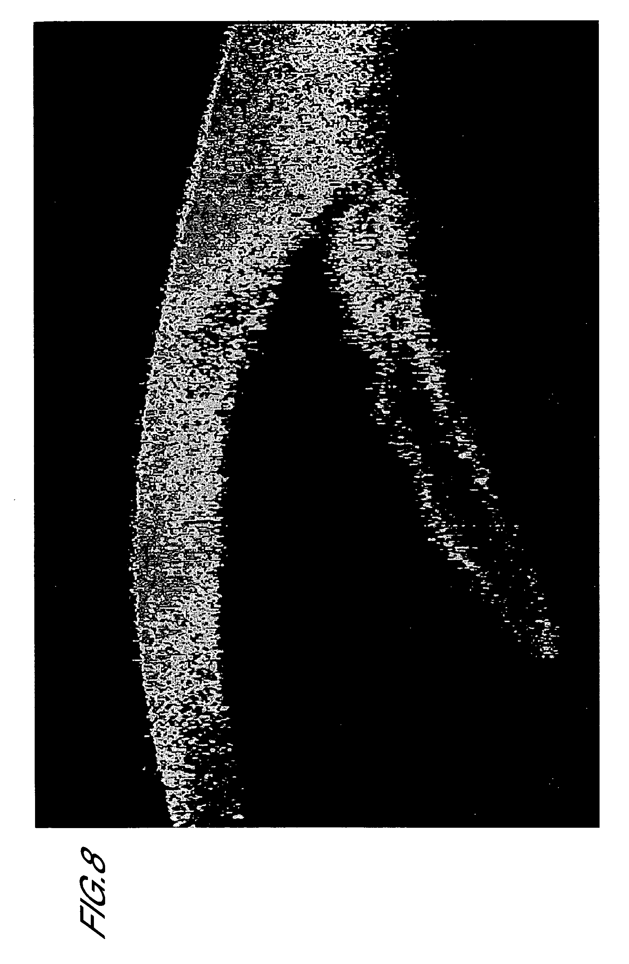

Fig. 8 is a tomographic image of an anterior eye portion captured separately. Even in the parts that are shaded by the sclera, the iris is clearly visible. It is believed that the reasons for being able to observe even the parts that are shaded by the sclera, which is a strong scatterer, so clearly are selection of a suitable measurement wavelength and the high sensitivity of the OFDR-OCT system. Note that the measurement speed can be increased easily, and by raising the wave number switching speed of the light source, measurement can be performed in 0.1 seconds or less.

Hence, according to this embodiment, the shape of the entire vicinity of the corner angle, which is constituted by the sclera, the cornea, and the iris, can be measured sufficiently with a clear tomographic image.

Further, in this embodiment, a Mach-Zehnder interferometer is constructed using the optical circulator 15, and thus the second coupler 13 and third coupler 16 are used, but by constructing a Michelson interferometer, main splitting/combining means doubling as main splitting means and combining means may be applied.

Further, in this embodiment the optical circulator 15 is applied, but in a case where the optical circulator 15 is not operated by the visible light produced by the aiming light source, for example, a coupler may be applied in place of the optical circulator 15, for example.

Also in this embodiment, the measuring head 40 is applied using the optical circulator 15 such that exit guidance of the measuring light and entrance guidance of the signal light can be implemented on the same optical path, but by omitting the optical circulator 15 and providing two parallel optical fibers in the interior of the main body tube 41 of the measuring head 40, exit guidance of the measuring light can be performed by one of the optical fibers, and entrance guidance of the signal light can be performed by the other optical fiber.

Note that at this time, the optical axes of the two optical fibers deviate slightly from each other such that a difference occurs between the optical axes of the exiting measuring light and the entering signal light, but this poses no practical problems.

Also in this embodiment, OFDR-OCT was described, but OCDR-OCT or FD-OCT may also be applied. A chirp OCT method (see Non-Patent Document 4 and so on, for example) may also be applied as a special example. The present invention may also be applied to OFDR-OCT in which the wavelength of the light source is swept continuously. When these other OCT methods are applied, the various instruments may be modified according to necessity in response to the respective features of each method.

For example, when the present invention is applied to the OCDR-OCT method, an SLD capable of emitting measuring light having a center wavelength of 1.55$\mu$m or 1.69$\mu$m and an emission spectrum width of 30nm may be used as the light source, and a Michelson interferometer (see Fig. 4 of Non-Patent Document 6 and so on, for example) may be used in the interference system. Other means, such as measuring light emitting means, are similar to those of this embodiment, while means for constructing a tomographic image may be constituted by a photodiode (PD), a personal computer (PC), and so on, such as those described in Fig. 4 of Non-Patent Document 6, for example.

Meanwhile, when the present invention is applied to the chirp OCT method, the wavelength tunable light source 11 of this embodiment may be replaced by a source which sweeps the wavelength continuously so that measurement can be performed while sweeping the wavelength continuously.

Incidentally, in the chirp OCT method, depth direction is obtained by subjecting the intensity of interference light, which is produced by varying the wavelength of the light source linearly relative to time, to Fourier transform on the temporal axis and detecting the frequency of the beat signal. In contrast, when the intensity of interference light obtained in the same manner is subjected to Fourier transform relative to the wave number rather than time, similar information to that obtained with the OFDR-OCT method can be detected. In this case, a problem occurs in that as the optical path length difference between a sample optical path (the optical path on which the measurement subject exists) and a reference optical path (another optical path) increases, the strength of the interference signal corresponding to each optical path length difference decreases. This phenomenon occurs because the wave number is not fixed, but varies gradually during sampling of the interference signal strength (the output of the second differential amplifier 18). However, it is not impossible to capture a tomographic image in this case, and therefore the present invention may also be applied to this method.

INDUSTRIAL APPLICABILITY

With the tissue measuring optical coherence tomography-use light source and tissue measuring optical coherence tomography system according to the present invention, an eye examination can be performed easily, for example, and therefore producing these systems is useful to the precision instrument manufacturing industry and so on.

**Claims**

1. A tissue measuring optical coherence tomography-use light source **characterized in** being capable of emitting light in a wavelength region of 1.53 to 1.85$\mu$m.

2. The tissue measuring optical coherence tomography-use light source according to claim 1, **characterized in** being capable of emitting said light while switching a wavelength of said light.

3. A tissue measuring optical coherence tomography-use light source **characterized in** being capable of emitting light having an coherence length in air of 1.4mm or more while switching a wavelength of said light discontinuously in a wave number interval of up to $1.2 \times 10^{-3}\mu m^{-1}$.

4. The tissue measuring optical coherence tomography-use light source according to claim 2, **characterized in that** said coherence length in air of said light is 1.4mm or more, and in being capable of switching said wavelength of said light discontinuously in a wave number interval of up to $1.2 \times 10^{-3} \mu m^{-1}$.

5. The tissue measuring optical coherence tomography-use light source according to any of claims 2 through 4, **characterized in** being capable of emitting said light while switching said wavelength of said light stepwise.

6. The tissue measuring optical coherence tomography-use light source according to any of claims 2 through 5, **characterized in that** a light source for producing said light is a wavelength tunable semiconductor laser.

7. The tissue measuring optical coherence tomography-use light source according to claim 6, **characterized in that** said wavelength tunable semiconductor laser is one of a superstructure grating distributed Bragg reflector semiconductor laser, a sampled grating distributed Bragg reflector semiconductor laser, and a grating coupler sampled reflector laser.

8. A tissue measuring optical coherence tomography system **characterized in** comprising the tissue measuring optical coherence tomography-use light source according to claim 1.

9. A tissue measuring optical coherence tomography system **characterized in** comprising:

 wavelength tunable light producing means comprising the tissue measuring optical coherence tomography-use light source according to any of claims 2 through 7;
 main splitting means for splitting light produced by said wavelength tunable light producing means into measuring light and reference light;
 measuring light illuminating means for illuminating said measuring light split by said main splitting means onto a target tissue while scanning said tissue;
 signal light collecting means for collecting signal light illuminated onto said tissue and reflected or backscattered thereby;
 combining means for combining said signal light collected by said signal light collecting means and said reference light split by said main splitting means; and
 calculation control means for controlling said wavelength tunable light producing means such that said light is produced by said wavelength tunable light producing means at a target wavelength, and determining a tomographic image of said tissue on the basis of said wavelength of said light produced by said wavelength tunable light producing means and an intensity of said light combined by said combining means.

10. The tissue measuring optical coherence tomography system according to claim 9, **characterized in that** said main splitting means and said combining means serve together as main splitting/combining means.

11. The tissue measuring optical coherence tomography system according to claim 9 or claim 10, **characterized in that** said measuring light illuminating means and said signal light collecting means serve together as illuminating/collecting means.

12. The tissue measuring optical coherence tomography system according to any of claims 8 through 11, **characterized in that** said tissue has a water content of at least 60% by weight.

13. The tissue measuring optical coherence tomography system according to claim 12, **characterized in that** said tissue is an eye.

14. The tissue measuring optical coherence tomography system according to claim 13, **characterized in that** an anterior eye portion of said eye is measured.

15. The tissue measuring optical coherence tomography system according to claim 13 or claim 14, **characterized in** comprising fixing/supporting means for fixedly supporting a face of a test subject while said test subject is in a sitting position and said eye remains oriented in a horizontal direction.

16. A tissue measuring optical coherence tomography system not comprising fixing/supporting means for fixedly supporting a face of a test subject while said test subject is in a sitting position and an eye remains oriented in a horizontal direction,
**characterized in** comprising means for attaching means for illuminating said eye with measuring light to a slit lamp microscope.

**17.** The tissue measuring optical coherence tomography system according to claim 13 or claim 14, **characterized in** not comprising fixing/supporting means for fixedly supporting a face of a test subject while said test subject is in a sitting position and said eye remains oriented in a horizontal direction, and comprising means for attaching means for illuminating said eye with measuring light to a slit lamp microscope.

**18.** An operating method for a tissue measuring optical coherence tomography system, **characterized in** comprising the steps of:

emitting light from wavelength tunable light producing means comprising the tissue measuring optical coherence tomography-use light source according to any of claims 1 through 7 while tuning a wavelength of said light;
performing main splitting to split said light produced by said wavelength tunable light producing means into measuring light and reference light;
illuminating said measuring light split by said main splitting means onto a target tissue while scanning said tissue;
collecting signal light illuminated onto said tissue and reflected or backscattered thereby;
combining said signal light collected by said signal light collecting means and said reference light split by said main splitting means; and
controlling said wavelength tunable light producing means such that said light is produced by said wavelength tunable light producing means at a target wavelength, and determining a tomographic image of said tissue on the basis of said wavelength of said light produced by said wavelength tunable light producing means and an intensity of said light combined by said combining means.

**19.** An eye diagnosing method which, when diagnosing an eye using a tissue measuring optical coherence tomography system having the tissue measuring optical coherence tomography-use light source according to any one of claims 1 through 7 and signal light collecting means for collecting signal light illuminated onto eye tissue and reflected or backscattered thereby, comprises the steps of:

illuminating tissue constituting said eye with light produced by said optical coherence tomography-use light source;
detecting reflection light or backscattered light generated in the interior of said tissue constituting said eye using said signal light collecting means; and
visually displaying a depth direction structure of said tissue constituting said eye on the basis of detection data detected by said detector.

*FIG.1*

*FIG.2*

FIG.3(a)

TIME

FIG.3(b)

TIME

FIG.3(c)

TIME

FIG.4

EP 1 787 588 A1

*FIG.5*

WAVELENGTH

FIG.6

## FIG.7

FIG.8

EP 1 787 588 A1

*FIG.9*

## INTERNATIONAL SEARCH REPORT

| | International application No. |
|---|---|
| | PCT/JP2005/015457 |

**A. CLASSIFICATION OF SUBJECT MATTER**
*A61B10/00* (2006.01)

According to International Patent Classification (IPC) or to both national classification and IPC

**B. FIELDS SEARCHED**

Minimum documentation searched (classification system followed by classification symbols)
*A61B10/00* (2006.01)

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>Y<br>A | JP 2002-95663 A  (Fuji Photo Film Co., Ltd.),<br>02 April, 2002 (02.04.02),<br>Claim 7<br>& US 2002/37252 A1 | 1,8<br>2-7,9-14<br>15-17 |
| X<br>Y<br>A | JP 2002-113017 A  (Fuji Photo Film Co., Ltd.),<br>16 April, 2002 (16.04.02),<br>Claim 6<br>(Family: none) | 1,8<br>2-7,9-14<br>15-17 |
| X<br>Y<br>A | JP 2002-214127 A  (Massachusetts Institute of Technology),<br>31 July, 2002 (31.07.02),<br>& US 5748598 A          & EP 883793 A1 | 1,8<br>2-7,9-14<br>15-17 |

| ☒ | Further documents are listed in the continuation of Box C. | ☐ | See patent family annex. |
|---|---|---|---|

| | | |
|---|---|---|
| * | Special categories of cited documents: | |
| "A" | document defining the general state of the art which is not considered to be of particular relevance | "T" later document published after the international filing date or priority date and not in conflict with the application but cited to understand the principle or theory underlying the invention |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| 01 November, 2005 (01.11.05) | 15 November, 2005 (15.11.05) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**

| International application No. |
|---|
| PCT/JP2005/015457 |

C (Continuation).    DOCUMENTS CONSIDERED TO BE RELEVANT

| Category* | Citation of document, with indication, where appropriate, of the relevant passages | Relevant to claim No. |
|---|---|---|
| Y | JP 2004-167080 A  (Kimiya SHIMIZU),<br>17 June, 2004 (17.06.04),<br>(Family: none) | 2-7,9-14 |
| Y | JP 11-108763 A  (Kabushiki Kaisha Seitaiko<br>Joho Kenkyusho),<br>23 April, 1999 (23.04.99),<br>(Family: none) | 2-7,9-14 |
| Y | JP 9-196812 A  (ATR Optical and Radio<br>Communications),<br>31 July, 1997 (31.07.97),<br>(Family: none) | 2-7,9-14 |
| A | JP 11-326182 A  (Kao Corp.),<br>26 November, 1999 (26.11.99),<br>(Family: none) | 15-17 |
| A | JP 2004-33277 A  (Nidek Co., Ltd.),<br>05 February, 2004 (05.02.04),<br>(Family: none) | 15-17 |

Form PCT/ISA/210 (continuation of second sheet) (April 2005)

## INTERNATIONAL SEARCH REPORT

| International application No. |
|---|
| PCT/JP2005/015457 |

---

**Box No. II     Observations where certain claims were found unsearchable (Continuation of item 2 of first sheet)**

This international search report has not been established in respect of certain claims under Article 17(2)(a) for the following reasons:

1. ☒ Claims Nos.: 18, 19
   because they relate to subject matter not required to be searched by this Authority, namely:
   Judging from facts that claims 18, 19 are provided with the step of applying a light to a tissue and the step of measuring a signal light reflected or rear-scattered after being applied to the tissue, these claims substantially pertain to diagnostic methods.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

---

**Box No. III     Observations where unity of invention is lacking (Continuation of item 3 of first sheet)**

This International Searching Authority found multiple inventions in this international application, as follows:
   There exists no special technical feature common to claim 1, claim 3, claim 16 that are independent claims. Claims 1, 8 are not novel nor involve an inventive step according to document (JP 2002-95663 A), document (JP 2002-113017 A), document (JP 2002-214127 A).
   Consequently, when main invention is classified as those portions, sharing the constitution of claim 4, of claims 1, 2, 4, 8 and claims 5-7, 9-14 and second and subsequent inventions are classified similarly, the claims in this application are considered to describe six inventions.
   Therefore, claims 1-17 do not fulfill the unity of invention.

1. ☒ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**     ☐ The additional search fees were accompanied by the applicant's protest and, where applicable, the payment of a protest fee..

☐ The additional search fees were accompanied by the applicant's protest but the applicable protest fee was not paid within the time limit specified in the invitation.

☒ No protest accompanied the payment of additional search fees.

Form PCT/ISA/210 (continuation of first sheet (2)) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4896325 A

### Non-patent literature cited in the description

- Handbook of Optical Coherence Tomography. Marcel Dekker Inc, 2002, 498-500
- Microscopic diagnostics using optical coherence tomography for clinical applications. **CHAN KIN PUI.** Optronics. Optronics Corp, 10 July 2002, 179-183
- Developmental trends of wavelength tunable lasers and expectations for system applications. **YUZO YOSHIKUNI.** Oyo Buturi. Applied Physics Scientific Society, 2002, vol. 71, 1362-1366
- Handbook of Optical Coherence Tomography. Marcel Dekker Inc, 364-367
- High speed, high resolution OFDR-OCT using SSG-DBR laser. **CHOI DONG HAK et al.** 28th Optical Symposium Lecture Handbook. The Optical Society of Japan, 19 June 2003, 39-40
- **MASAMITSU HARUNA.** Tissue measuring and tomographic imaging using low coherent optical interference. *Oyo Kogaku,* 2003, vol. 2, 1-6